# EUROPÄISCHE PATENTSCHRIFT

(11) **EP 2 925 852 B1**
(45) Veröffentlichungstag und Bekanntmachung des Hinweises auf die Patenterteilung: **06.06.2018**
(21) Anmeldenummer: 13776518.6
(22) Anmeldetag: 15.10.2013
(51) Int. Cl.: C12M 1/107, C12M 1/16, C12M 1/34, C12M 1/36

(54) **VERFAHREN UND VORRICHTUNG ZUR ERZEUGUNG VON BIOGAS**
METHOD AND DEVICE FOR PRODUCING BIOGAS
PROCÉDÉ ET DISPOSITIF DE PRODUCTION DE BIOGAZ

(30) Priorität: 15.10.2012 DE 102012109822
(43) Veröffentlichungstag der Anmeldung: 07.10.2015
(73) Patentinhaber: Renergon International AG, 8574 Lengwil (CH)
(72) Erfinder: RESTLE, Karl-Heinz, CH-8280 Kreuzlingen (CH); ZAK, Manuel, 78467 Konstanz (DE)
(74) Vertreter: Nordmeyer, Philipp Werner
(86) Internationale Anmeldenummer: PCT/EP2013/071542
(87) Internationale Veröffentlichungsnummer: WO 2014/060423

(56) Entgegenhaltungen:
- EP-A1- 1 997 875
- EP-A2- 2 270 127
- DE-A1- 10 354 406

## Beschreibung

### Technisches Gebiet

Die vorliegende Erfindung betrifft ein Verfahren und eine Vorrichtung zur Erzeugung von Biogas aus organischen Feststoffen, bevorzugt aus stapelbarer Biomasse.

### Stand der Technik

Es ist bekannt, organische Feststoffe und insbesondere stapelbare Biomasse in so genannten Feststofffermentern zu vergären, um hieraus Biogas zu gewinnen. Das Biogas wird nachfolgend üblicherweise zur Wärmeerzeugung und/oder über Blockheizkraftwerke zur gekoppelten Strom- und Wärmegewinnung verwertet. Nach einer Aufreinigung kann das Biogas auch als Biomethan vertrieben werden und kann beispielsweise in das Erdgasnetz eingespeist oder zum Betrieb von Fahrzeugen verwendet werden.

In der bekannten Feststofffermentation werden die organischen Feststoffe in einen Feststofffermenter eingebracht und dann periodisch mit einem Perkolatstrom beaufschlagt. Das durch die stapelbare Biomasse gebildete Feststoffbett hindurchgerieselte Perkolat wird üblicherweise am Boden des Feststofffermenters gesammelt, in einem Kreislauf geführt und dann wieder über der stapelbaren Biomasse in dem jeweiligen oder einem anderen Feststofffermenter verrieselt. Der Perkolatstrom wird von der Decke des Feststofffermenters aus auf die Biomasse aufgebracht, um den Gärprozess zu starten und danach in Gang zu halten.

Der Feststofffermenter ist üblicherweise garagenartig ausgebildet und weist gasdichte Wände, Decke und Boden auf, welche häufig aus Beton gegossen sind, sowie ein gasdichtes Tor. Auf diese Weise wird eine anaerobe Vergärung der Biomasse möglich. Das Biogas wird üblicherweise über entsprechende Leitungsvorrichtungen aus den Feststofffermentern abgezogen. Durch das Tor kann der Feststofffermenter mit den organischen Feststoffen beladen werden.

Nachdem der Vergärprozess einer Beladung eines Feststofffermenters mit organischen Feststoffen abgeschlossen ist, also ungefähr nach 4-5 Wochen, wird der Gärrest durch das Tor aus dem entsprechenden Feststofffermenter ausgetragen.

In den bekannten Verfahren werden die organischen Feststoffe in einem anaeroben Milieu im Feststofffermenter vergoren, wobei die entsprechend benötigten Mikroorganismen unter anderem mittels des aufgerieselten Perkolats zugeführt werden. Die organischen Feststoffe werden entsprechend durch das Perkolat "geimpft". Im Feststofffermenter werden die organischen Feststoffe dann unter Bildung von Biogas vergoren.

Der Vergärungsprozess kann in die folgenden vier Stufen unterteilt werden: In der ersten Stufe, der Hydrolyse, werden die in der organischen Biomasse vorhandenen Biomoleküle durch Reaktion mit Wasser in Bruchstücke gespalten, wobei Wasserstoff und CO₂ freigesetzt wird. In der zweiten Stufe, der Acidogenese, werden die bei der Hydrolyse entstandenen Bruchstücke einerseits in niedere Fettsäuren und/oder Karbonsäuren wie z.B. Buttersäure oder Propionsäure, andererseits in niedere Alkohole wie z.B. Ethanol, umgesetzt. In der dritten Stufe, der Acetogenese, werden die während der Hydrolyse und Acidogenese gebildeten niederen Fettsäuren und/oder Karbonsäuren sowie die niederen Alkohole durch acetogene Mikroorganismen primär zu Essigsäure, beziehungsweise zu dessen gelöstem Salz, dem Acetat, umgesetzt. In der vierten Stufe, der Methanogenese, wird auf der einen SeiteEssigsäure durch Essigsäure-spaltende Methanbildner in Methan und Kohlenstoffdioxid umgewandelt und auf der anderen Seite ensteht Methan aus Wasserstoff und CO₂ durch eine andere Gruppe von Methanbildnern. Das aus diesem Prozess entstehende Biogas setzt sich zum großen Teil aus Methan zusammen. Es handelt sich entsprechend um ein energiereiches Gas, welches unter den anoxischen Bedingungen aus den organischen Feststoffen durch Vergärung entsteht.

Aus der DE 10 2005 029 306 B4 ist ein Verfahren zum Betreiben einer Feststofffermenteranlage sowie eine Vorrichtung hierzu bekannt. Gemäß der Lehre dieses Dokuments wird der Perkolatstrom für jeden vorgesehenen Feststofffermenter so gesteuert, dass die in den Feststofffermentern entstehende Säure mit dem Perkolatstrom so weit ausgeschwemmt wird, dass keine Versäuerung der in dem Feststofffermenter gelagerten Biomasse eintritt und gleichzeitig der Perkolatstrom eine solche Beladung an Mikroorganismen aufweist, dass er zum Starten der Fermentation in einem neu angesetzten Feststofffermenter einsetzbar ist. Dabei wird sowohl den aktiven Feststofffermentern als auch einem kontinuierlich arbeitenden Regenerationsfermenter für das Perkolat, welcher als Perkolatbehälter dient, Biogas entnommen.

Aus der WO 02/06439 A2 ist ein Bioreaktor zur Methanisierung von Biomasse und eine Biogasanlage zum Erzeugen von thermischer, elektrischer oder mechanischer Energie aus Biomasse mit einem entsprechenden Bioreaktor bekannt. In dieser Anmeldung wird beschrieben, dass die aus den als Boxenfermenter ausgebildeten Biogasreaktoren austretende Sickersäfte bezüglich ihres pH-Werts, ihres Nährstoffgehaltes und anderer wichtiger Parameter vermessen werden. Den Sickersäften werden dann Zusatzstoffe beigefügt und die derart aufbereiteten Sickersäfte werden als Perkolat verwendet.

Aus der EP 2 270 127 A2 ist ein Bioreaktor zur Methanisierung von Biomasse mit hohem Feststoffanteil bekannt. Darin ist beschrieben, dass Sickersäfte in einem Faulbehälter des Bioreaktors bis zu einem bestimmten Flüssigkeitspegel aufgestaut werden. Dieser Flüssigkeitspegel kann eingestellt oder geregelt werden, um die Biogaserzeugungsrate oder die Biogasausbeute zu maximieren.

### Darstellung der Erfindung

Ausgehend von dem bekannten Stand der Technik ist es eine Aufgabe der vorliegenden Erfindung, die Gasausbeute in einem Feststofffermenter weiter zu optimieren, um die Anlagenkapazitäten und die zu vergärende Biomasse optimal ausnutzen zu können.

Diese Aufgabe wird durch ein Verfahren zur Erzeugung von Biogas in einem Feststofffermenter mit den Merkmalen des Anspruchs 1 gelöst. Vorteilhafte Weiterbildungen ergeben sich aus den Unteransprüchen.

Entsprechend wird ein Verfahren zur Erzeugung von Biogas in einem Feststofffermenter, in welchem stapelbare Biomasse von einem Perkolatstrom durchströmbar ist, um in einem Vergärprozess Biogas zu erzeugen, vorgeschlagen. Erfindungsgemäß wird der Vergärprozess anhand der Qualität des erzeugten Biogases geregelt.

Dadurch, dass der Vergärprozess anhand der Qualität des erzeugten Biogases geregelt wird, kann der Ertrag an verwertbarem Biogas optimiert werden. Im Gegensatz zu den aus dem Stand der Technik bekannten Steuerungen, welche die Betriebsparameter eines Feststofffermenters anhand vorgegebener, weitgehend starrer Muster steuern, kann über die vorgeschlagene Regelung und den damit einhergehenden Rückkopplungsmechanismus erreicht werden, dass zu jedem gegebenen Zeitpunkt bzw. in jedem gegebenen Zeitabschnitt der Feststofffermenter in einem optimierten Bereich betrieben wird.

Über eine Regelung der erzeugten Biogasqualität beispielweise auf einen vorgegebenen Methangasanteil hin kann über einen längeren Zeitraum hinweg eine konstante Ausbeute an Methangas erreicht werden, wodurch die nachfolgende Verwertung vereinfacht wird. Beispielsweise wird die Verbrennung in einem nachgeschalteten Blockheizkraftwerk (BHKW) vereinfacht, wenn ein konstanter Methangasanteil im zugeführten Biogas vorliegt.

In der Startphase eines Feststofffermenters, beispielsweise direkt nach dem Schließen des gasdichten Tores, kann über die Regelung ein schneller Beginn des Vergärprozesses erreicht werden, da die Qualität des abgezogenen Biogases als Regelgröße verwendet wird. Entsprechend können in der Startphase des Vergärprozesses die Betriebsparameter des Feststofffermenters so geregelt werden, dass das abgezogene Biogas einem vorgegebenen Qualitätsverlauf folgt. Damit kann auch in der Startphase des Vergärungsprozesses unmittelbar auf unterschiedliche Beladungen des Feststofffermenters eingegangen werden, ohne dass die spezifische Zusammensetzung der Beladung bekannt sein muss. Vielmehr wird der Vergärprozess durch die Variation der Betriebsparameter in einem derart optimierten Bereich betrieben, dass das abgezogene Biogas einem vorgegebenen Soll-Verlauf der Regelgröße folgt.

Damit wird als Regelgröße die Biogasqualität verwendet, also beispielsweise das erzeugte Biogasvolumen beziehungsweise der Biogasvolumenstrom, der Methangasgehalt und/oder der Restgasgehalt, und die Soll-Regelgröße wird über eine Führgröße entlang des optimalen dynamischen Verlaufs des Vergärvorganges geführt.

Die Führgröße kann entweder für einen gegebenen Feststofffermenter vorgegeben sein, oder aber aufgrund weiterer Parameter, wie beispielsweise der Zusammensetzung der jeweils in den Feststofffermenter eingebrachten Biomasse, der bisherigen Verweildauer, der Menge der eingebrachten Biomasse und/oder des aktuellen Vergärzustandes gebildet werden, um hier eine weitere Optimierung des Vergärvorganges durchzuführen.

Auf diese Weise kann weiterhin im Zusammenspiel mit den anderen Feststofffermentern einer Biogasanlage eine gleichbleibende Biogasqualität erreicht werden, da die einzelnen Feststofffermenter einem vorgegebenen Verlauf in der Biogasqualität folgen und entsprechend das aus allen aktiven Feststofffermentern zusammen gemischte Biogas im Zeitverlauf stets eine gleichbleibende Qualität aufweisen kann.

Hierbei ist zu berücksichtigen, dass die in einen Feststofffermenter einbringbare stapelbare Biomasse üblicherweise von Beladung zu Beladung bezüglich ihrer Zusammensetzung, ihrer Porosität, ihres Energiegehalts, ihres Faseranteils sowie ihres Feuchtegehalts variiert. Über die weitgehend starren Steuermuster des Standes der Technik kann diesen unterschiedlichen Gegebenheiten nicht Rechnung getragen werden. Durch die vorgeschlagene Regelung, welche als Regelgröße die Gasqualität verwendet, kann jedoch unabhängig von der jeweiligen Beladung des Feststofffermenters die Gasausbeute optimiert werden.

Weiterhin handelt es sich bei der Vergärung von stapelbarer Biomasse in einem Feststofffermenter um einen biologischen Prozess, welcher nicht vollständig vorhersagbar und entsprechend nicht über ein starres Steuermuster optimal ausbeutbar ist. Über die vorgeschlagene Regelung können auch diese Abweichungen im Vergärprozess, welche von Feststofffermenter zu Feststofffermenter in einer Biogasanlage unterschiedlich sein können, sowie welche von Beladung von Beladung variieren können, berücksichtigt werden, und es wird dennoch stets ein optimierter Vergärprozess erreicht.

Darüber hinaus kann auch die Zusammensetzung des Perkolats, welches zur Berieselung der stapelbaren Biomasse in dem Feststofffermenter vorgesehen ist, variieren, beispielsweise bezüglich seiner Beladung mit Mikroorganismen, welche schlussendlich zur Ausbildung der das Biogas erzeugenden Biofilme in der Biomasse führt.

Unter der Qualität des erzeugten Biogases werden das erzeugte Biogasvolumen, der erzeugte Biogasvolumenstrom, der Methangasgehalt und/oder der Restgasanteil in dem erzeugten Biogas verstanden. Andere Parameter in dem erzeugten Biogas können ebenfalls als Regelgröße zur Regelung des Vergärprozesses in dem Feststofffermenter hinzugezogen werden, so wie beispielsweise die Variation des Biogasvolumenstroms oder des Methangasgehalts.

Die Regelung des Vergärprozesses wird darüber durchgeführt, dass der Feststofffermenter über die Regelung mindestens eines Betriebsparameters des Feststofffermenters geregelt wird. Hier sind unterschiedliche Betriebsparameter direkt zugänglich, so wie die Temperatur im Feststofffermenter, welche über die Perkolattemperatur geregelt werden kann, die Frequenz der Perkolierung, die Perkolierungsdauer, das jeweils in einem einzelnen Perkolierungsvorgang aufgebrachte Perkolatvolumen, die Temperatur des Perkolats, der pH-Wert des Perkolats und/oder andere direkt zugängliche Parameter, welche als Stellgrößen in der vorgeschlagenen Regelung verwendet werden können.

In einer weiteren bevorzugten Ausbildung des Verfahrens wird die Verweildauer der stapelbaren Biomasse im Feststofffermenter ebenfalls anhand der Qualität des erzeugten Biogases geregelt. Mit anderen Worten wird der Öffnungszeitpunkt des Feststofffermenters anhand der Qualität des erzeugten Biogases geregelt, so dass für unterschiedliche Feststofffermenter innerhalb einer Biogasanlage unterschiedliche Verweildauern der jeweiligen stapelbaren Biomasse erreicht werden können.

In einer besonders bevorzugten Variante wird die Zusammensetzung, die Menge, die bisherige Verweildauer und/oder der Vergärzustand der im Feststofffermenter aufgenommenen Biomasse bei der jeweiligen Regelung berücksichtigt. Bevorzugt wird die Soll-Regelgröße beziehungsweise die Führungsgröße zur dynamischen Nachführung der Soll-Regelungsgröße unter Berücksichtigung der genannten Parameter bestimmt.

Die Regelung des jeweiligen Vergärprozesses anhand der Qualität des erzeugten Biogases findet für jeden individuellen Feststofffermenter einer Biogasanlage separat statt. Entsprechend kann der Verlauf einer jeweiligen Vergärung innerhalb eines Feststofffermenters für zwei Feststofffermenter innerhalb einer Biogasanlage vollkommen unterschiedlich verlaufen, abhängig davon, mit welchem Biomassenmix die jeweiligen Feststofffermenter beladen wurden, und welche anderen äußeren Parameter in den jeweiligen Vergärprozess eingreifen. Die beiden unterschiedlichen Verläufe der Vergärung in den jeweiligen Feststofffermentern führen jedoch in beiden Fällen zu einer Optimierung der Ausbeute an Biogas und insbesondere zu einer Optimierung der Ausbeute an Methan in dem jeweiligen Vergärungsprozess, so dass nach Abschluss des Vergärprozesses die bestmögliche Biogasproduktion erreicht wurde.

Besonders bevorzugt wird die Perkolierung anhand der Biogasqualität geregelt, bevorzugt durch Regelung der Perkolationsdauer, der aufgebrachten Perkolatmenge pro Perkolatsvorgang und/oder der Perkolationsfrequenz.

In einer weiteren bevorzugten Ausgestaltung des Verfahrens werden mindestens zwei Feststofffermenter gleichzeitig aufgrund der Qualität des miteinander vermischten Gasstroms aller Biogasströme geregelt. So kann der aus einer Biogasanlage ausgestoßene Biogasertrag bezüglich der Qualität des Biogases geregelt werden, um entsprechend aus dem Wechselspiel der individuellen Feststofffermenter eine konstante Biogasqualität zu erreichen.

Die oben gestellte Aufgabe wird weiterhin durch ein Verfahren zur Erzeugung von Biogas in einem Feststofffermenter, in welchem stapelbare Biomasse mit Perkolat beaufschlagt wird, um in einem Vergärprozess Biogas zu erzeugen, mit den Merkmalen des Anspruchs 7 gelöst. Erfindungsgemäß wird die Beaufschlagung der Biomasse mit Perkolat anhand der Qualität des erzeugten Biogases geregelt. Die Perkolationsdauer, die aufgebrachte Perkolatmenge und/oder die Perkolationsfrequenz werden anhand der Qualität des erzeugten Biogases geregelt, bevorzugt anhand des erzeugten Biogasvolumens, des erzeugten Biogasvolumenstroms, des Methangehalts und/oder des Restgasgehalts.

Weiterhin wird eine Vorrichtung zur Erzeugung von Biogas, umfassend mindestens einen Feststofffermenter zur Aufnahme stapelbarer Biomasse, wobei die stapelbare Biomasse von einem Perkolatstrom durchströmbar ist, um in einem Vergärprozess Biogas zu erzeugen, mit den Merkmalen des Anspruchs 8 vorgeschlagen. Erfindungsgemäß ist eine Regelung zur Regelung des Vergärprozesses anhand der Qualität des erzeugten Biogases vorgesehen.

Bevorzugt ist ein Sensor zur Bestimmung der Qualität des aus einem Feststofffermenter abgezogenen Biogases vorgesehen und die Regelung regelt die Perkolierung des Feststofffermenters.

Dabei wirkt die Regelung auf die Heiztemperatur des Perkolats, die Perkolationsdauer, die Perkolationsfrequenz, die Menge des aufgebrachten Perkolats und/oder die pH-Werts des Perkolats als Stellgröße.

### Kurze Beschreibung der Figuren

Bevorzugte weitere Ausführungsformen und Aspekte der vorliegenden Erfindung werden durch die nachfolgende Beschreibung der Figuren näher erläutert.
- Figur 1: zeigt schematisch eine Vorrichtung zur Erzeugung von Biogas mittels des beschriebenen Verfahrens;
- Figur 2: zeigt schematisch den Regelkreislauf in einer Biogasanlage; und
- Figur 3: zeigt schematisch den Verlauf der Biogasproduktion in einem Feststofffermenter.

### Detaillierte Beschreibung bevorzugter Ausführungsbeispiele

Im Folgenden werden bevorzugte Ausführungsbeispiele anhand der Figuren beschrieben. Dabei werden gleiche, ähnliche oder gleichwirkende Elemente in den unterschiedlichen Figuren mit identischen Bezugszeichen bezeichnet und auf eine wiederholte Beschreibung dieser Elemente wird teilweise verzichtet, um Redundanzen in der Beschreibung zu vermeiden.

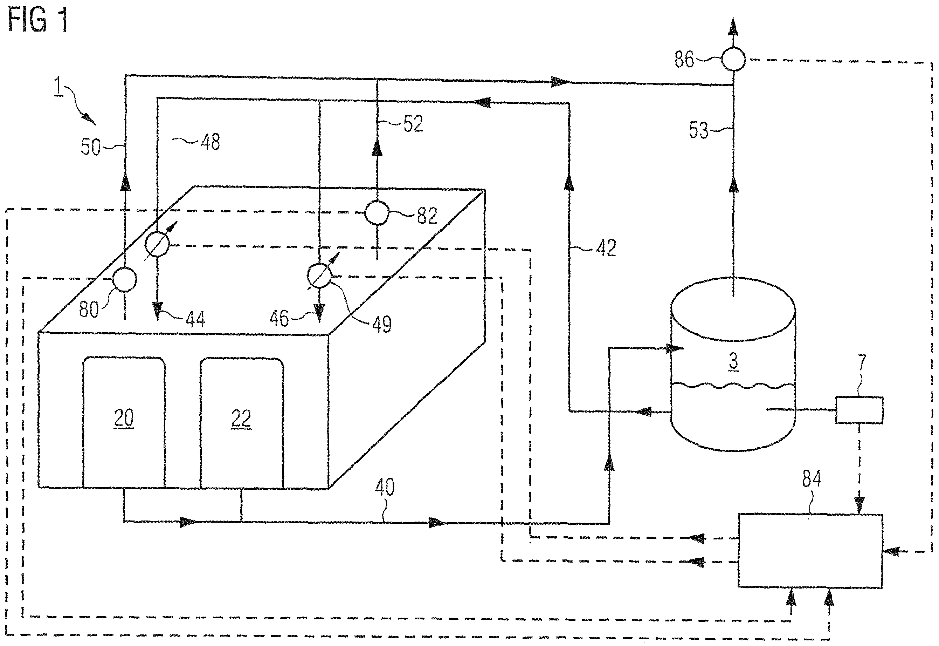

In Figur 1 ist eine Biogasanlage 1 zur Durchführung des Verfahrens zur Erzeugung von Biogas gezeigt.

Ein erster Feststofffermenter 20 und ein zweiter Feststofffermenter 22 sind exemplarisch in der Biogasanlage 1 vorgesehen, in welchen jeweils stapelbare Biomasse zur Vergärung aufgenommen werden kann. Üblicher Weise sind in einer Biogasanlage 1 eine größere Anzahl an Feststofffermentern vorgesehen, beispielsweise 5 bis 12. Die Feststofffermenter 20, 22 sind in bekannter Weise aufgebaut und weisen insbesondere gasdichte Wände, Böden und Decken auf, sowie ein gasdicht verschließendes Tor, durch welches der jeweilige Feststofffermenter 20, 22 mit organischen Feststoffen beschickt wird und durch welches hindurch der Gärrest nach Abschluss des Fermentationsvorganges wieder ausgeräumt wird.

Weiterhin ist ein Perkolattank 3 vorgesehen, in welchem Perkolat aufgenommen werden kann und welcher entsprechend als Perkolatspeicher dient. Das Perkolat wird über in den Feststofffermentern angeordnete Perkolatdüsen 44, 46 auf die Biomasse aufgebracht.

Aus dem Perkolattank 3 wird über die Perkolatleitung 42 Perkolat so in die jeweiligen Feststofffermenter 20, 22 geleitet, dass es über die an der Decke der Feststofffermenter 20, 22 vorgesehenen Perkolatdüsen 44, 46 auf die organischen Feststoffe, welche innerhalb der Feststofffermenter 20, 22 angeordnet sind, aufgerieselt werden kann. Das Perkolat sammelt sich am Boden der Feststofffermenter 20, 22, nachdem es die organischen Feststoffe durchlaufen hat. Daher sind im Bodenbereich der Feststofffermenter 20, 22 entsprechende Perkolatleitungen 40 vorgesehen, die mit dem Perkolattank 3 verbunden sind, so dass das auf dem Boden der jeweiligen Feststofffermenter 20, 22 gesammelte Perkolat über die Perkolatleitung 40 zurück in den Perkolattank 3 geleitet werden kann.

Entsprechend wird ein Perkolatkreislauf ausgebildet, wobei das Perkolat aus dem Perkolattank 3 über die Perkolatleitung 42 und die Perlkolatdüsen 44, 46 in die Feststofffermenter 20, 22 eingerieselt wird, und nach dem Durchlaufen des organischen Feststoffes am Boden der Feststofffermenter 20, 22 eingesammelt wird und über die Perkolatleitung 40 wieder dem Perkolattank 3 zugeführt wird.

Weiterhin sind Biogasleitungen 50 und 52 vorgesehen, mittels welchen das in den Feststofffermentern 20, 22 erzeugte Biogas abgezogen werden kann. Zusätzlich ist eine Biogasleitung 53 vorgesehen, mittels welcher das im Perkolattank 3 entstehende Biogas abgeleitet werden kann.

Zusätzliches Perkolatvolumen entsteht beispielsweise im Vergärungsprozess dadurch, dass bei der Vergärung Wasser produziert wird. Weiterhin werden über feuchte organische Feststoffe Zusatzvolumina in den Perkolatkreislauf eingetragen.

Das über die Biogasleitungen 50, 52, 53 abgeleitete Biogas wird nachfolgend entweder direkt in einem Blockheizkraftwerk (BHKW) in Strom und Wärme umgewandelt, oder aber es wird aufgereinigt und entsprechend als Biomethan in den Verkehr gebracht.

Die chemischen, physikalischen und/oder biologischen Eigenschaften des Perkolats im Perkolattank 3 werden mittels eines entsprechenden Sensors 7 gemessen. Der Sensor 7 kann beispielsweise den pH-Wert, die Temperatur, den FOS/TAC-Wert, und andere, für die Vergärung wesentliche Parameter messen.

In der schematischen Darstellung der Figur 1 sind in den Biogasleitungen 50, 52, welche das in den jeweiligen Feststofffermentern 20, 22 erzeugte Biogas austragen, Sensoren 80, 82 vorgesehen, welche die Qualität des erzeugten Biogases messen. Hier werden zum einen Volumensensoren eingesetzt, mittels welchen die gesamte, in einem bestimmten Feststofffermenter erzeugte Biogasmenge beziehungsweise der Volumenstrom bestimmt werden kann. Zum anderen können hier beispielsweise Sensoren 80, 82 eingesetzt werden, mittels welchen der Methangasanteil in dem erzeugten Biogas gemessen werden kann, oder aber es kann ein Restgasanteil bestimmt werden.

Auf der Grundlage der Messwerte der Biogasqualität kann entsprechend über eine entsprechende Regelung 84 beispielsweise die Perkolierung der jeweiligen Feststofffermenter 20, 22 geregelt werden. Hierzu können von der Regelung 84 entsprechend Ventile 48, 49 in den Perkolatleitungen 42 so angesteuert werden, dass über die Perkolatdüsen 44, 46 die vorbestimmte Perkolatmenge auf die Biomasse in dem jeweiligen Feststofffermenter 20, 22 aufgebracht wird.

Als Regelgröße in der Regelung wird dann der entsprechende Messwert des Sensors 80, 82 verwendet und mit einem vorgegebenen Sollwert der Regelgröße verglichen. Über eine entsprechende Anpassung eines oder mehrerer Betriebsparameter des Feststofffermenters 20, 22, welche als Stellgröße dienen, wird die Regelgröße wieder auf den Sollwert zurückgeführt. Wenn entsprechend beispielsweise ein Abfall in der Biogasproduktion und/oder im Methangasgehalt an einem Sensor 80, 82 detektiert wird, wird die Perkolationsfrequenz, die Perkolationsdauer oder die aufgebrachte Perkolatmenge in dem jeweiligen Feststofffermenter entsprechend über die Ventile 48, 49 so angepasst, dass die Biogasproduktion wieder auf den vorgegebenen Sollwert angehoben wird.

Da der Vergärprozess und damit die Biogasausbeute unterschiedliche Phasen durchläuft, muss auch der Sollwert der Regelgröße über den Vergärprozess hinweg angepasst werden, um eine optimale Ausnutzung der vorgehaltenen Anlagenkapazitäten und der eingesetzten Biomasse zu erreichen. Entsprechend wird eine Führungsgröße bestimmt auf Grundlage des Zeitverlaufs und bevorzugt unter Berücksichtigung der Zusammensetzung der Biomasse bestimmt. Die Führungsgröße bestimmt entsprechend den Verlauf des Sollwerts der Regelgröße. Beispielsweise steigt die Biogasausbeute zu Beginn des Vergärprozesses erst langsam an, was durch einen entsprechenden Verlauf der Führungsgröße abgebildet werden kann.

Zu Beginn des Vergärprozesses, wenn die noch trockene Biomasse zunächst angeimpft werden muss, kann beispielsweise über eine Messung der Gasqualität und eine daraus sich ergebende Regelung der Perkolierung ein optimiertes Startverhalten des jeweiligen Feststofffermenters 20, 22 erreicht werden. So kann es zu Beginn des Vergärprozesses sinnvoll sein, in kürzeren Intervallen zu Perkolieren, bei einer entsprechend angepassten Perkolatmenge, um eine gute Durchfeuchtung der frisch in den Fermenter eingebrachten Biomasse zu erreichen. Diese Anfangsphase einer mit kurzen Intervallen durchgeführten Perkolierung kann aufgrund des dann ansteigenden Methangasgehaltes in dem erzeugten Biogas auf eine Perkolierung mit längeren Intervallen und entsprechend ebenfalls angepasster Perkolatmenge zurückgeregelt werden. Der Zeitpunkt, wann dies geschieht, um eine optimale Biogasausbeute zu erhalten, kann über die Messung der Gasqualität bestimmt werden.

Es hat sich gezeigt, dass die Regelung des Vergärprozesses über die eigentlich interessierende Regelgröße, nämlich die Methanausbeute, eine Vielzahl anderer Steuermechanismen überflüssig macht.

Die von der Regelung 84 ausgegebene Steuergröße in Form der jeweiligen Veränderung der Betriebsparameter des Feststofffermenters 20, 22 führt entsprechend zu einer Regelung der Gasqualität. Eine Perkolierung der in einer oder beiden der Feststofffermentern 20, 22 angeordneten stapelbaren Biomasse wird dann entsprechend für eine vorgegebene Zeitdauer ausgelöst, bis die Regelgröße, also die Biogasqualität, wieder der Soll Regelgröße, also der gewünschten Biogasqualität, entspricht.

Die Verzögerung, welche sich durch das Durchsickern des Perkolats durch den organischen Feststoff sowie die Sammlung des Perkolats am Boden des Feststofffermenters ergibt, kann bereits von der Regelung 84 berücksichtigt werden.

In einer Weiterbildung oder einer Variante wird die Biogasqualität über einen Sensor 86 bestimmt, welcher in einem Leitungsabschnitt angeordnet ist, in welchem die Biogasströme aus allen

Feststofffermentern 20, 22 bereits miteinander vermischt sind. Dieser Sensor 86 misst entsprechend die Qualität des Biogases, welches schlussendlich aus der Biogasanlage 1 ausgegeben wird, beispielsweise, um es in einem Blockheizkraftwerk zu verwenden, oder welches der Weiterverarbeitung oder Aufbereitung zur Einspeisung in ein Gasnetz zugeführt wird. Die Regelung 84 kann entsprechend auch die Qualität des gesamten erzeugten Biogases als Regelgröße verwenden und die Betriebsparameter für die einzelnen Feststofffermenter 20, 22 so als Steuergrößen verwenden, dass eine möglichst gleichmäßige Biogasqualität aus der Biogasanlage 1 ausgegeben wird. Hierzu berücksichtigt die Regelung 84 auch die unterschiedlichen Vergärzustände in den individuellen Feststofffermentern 20, 22 und gibt entsprechend die Führungsgröße für den individuellen Feststofffermenter 20, 22 so vor, dass eine optimale Gasausbeute bei gleichzeitig gleichmäßiger Gasqualität erreicht wird.

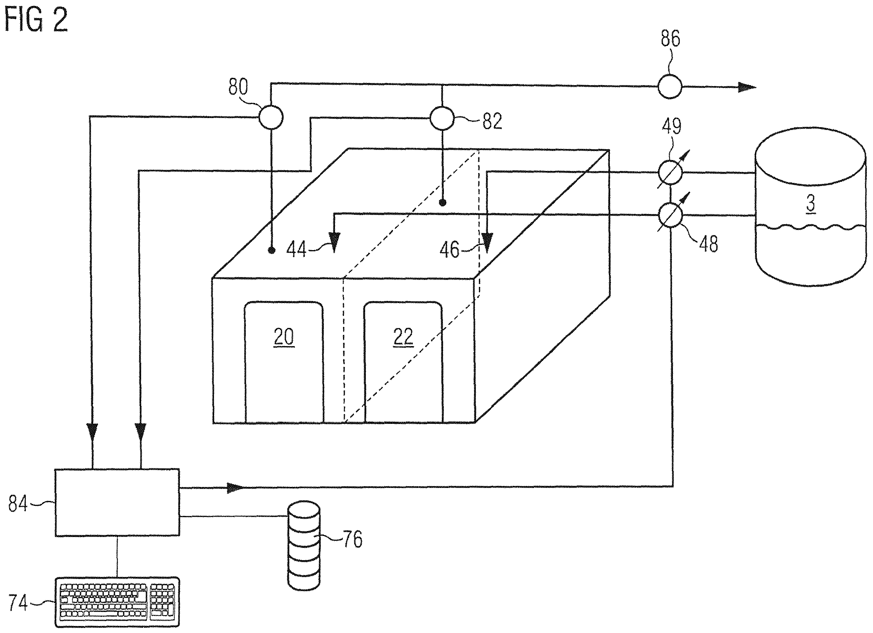

In Figur 2 ist schematisch noch einmal der Regelkreislauf gezeigt. Die beiden Feststofffermenter 20, 22 produzieren Biogas, dessen Qualität über die Sensoren 80, 82 gemessen wird. Hierbei werden besonders das erzeugte Biogasvolumen, der Methangasgehalt sowie möglicherweise der Gehalt an bestimmten Restgasen im Biogasstrom bestimmt. Die auf diese Weise erhaltenen Messwerte werden einer zentralen Regelung 84 zugeführt. Über die zentrale Regelung 84 wird dann die Zufuhr von Perkolat über Ventile 48, 49 in die jeweiligen Feststofffermenter 20, 22 geregelt. Beispielsweise werden über die Regelung 84 die Perkolationsdauer, die Perkolationsfrequenz sowie die Menge des jeweils aufgebrachten Perkolats auf das in den Feststofffermenter 20, 22 angeordnete Feststoffbett individuell für jeden Feststofffermenter separat geregelt.

Die Regelung 84 kann dabei zusätzlich noch mit Eingaben von Benutzern, welche beispielsweise über eine Tastatur 74 durchgeführt werden, über bestimmte Besonderheiten der jeweiligen Biomassebeladung in dem individuellen Feststofffermenter 20, 22 informiert werden. Beispielsweise kann auf eine bestimmte Substratzusammensetzung, beispielsweiche auch eine Einzelsubstratzusammensetzung, hingewiesen werden, welche möglicherweise ein verändertes Regelverhalten erfordern würde. Besonders bevorzugt kann auf diese Weise der Verlauf der Führungsgröße angepasst werden.

Über eine Datenbank 76 können entsprechende weitere Parameter abgefragt werden, welche für den jeweiligen individuellen Vergärprozess von Bedeutung sein können.

Die eigentliche Perkolierung wird jedoch über die Messung der Qualität des erzeugten Biogases geregelt.

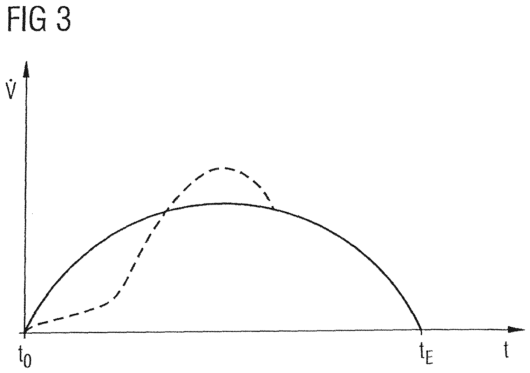

Figur 3 zeigt schematisch den typischen Verlauf eines Vergärungsprozesses eines Feststofffermenters über die Zeit hinweg vom Schließzeitpunkt t₀ des Feststofffermenters nach der Neubeladung mit stapelbarer Biomasse, bis hin zum Endzeitpunkt t_{E}, an welchem der Vergärungsprozess beendet wird, da sämtliche Biomasse umgesetzt ist. Auf der Ordinate ist der erzeugte Biogasstrom aufgetragen. Der mit der durchgezogenen Linie gezeigte Verlauf entspricht einem optimalen Verlauf des Biogasstroms in einem Feststofffermenter. Der mit der durchgezogenen Linie gezeigte Verlauf entspricht damit dem Verlauf des Sollwerts der Regelgröße beziehungsweise entsprechend der Führungsgröße in dem vorgeschlagenen Verfahren.

Die gestrichelt dargestellten Verläufe sind Abweichungen von dem Sollverlauf und werden durch die entsprechende Regelung der jeweiligen Betriebsparameter auf den Sollwert zurückgeführt. In einer optimalen Verfahrensführung wird die Produktion von Biogas entsprechend anhand des Sollwertverlaufs geregelt.

Soweit anwendbar können alle einzelnen Merkmale, die in den einzelnen Ausführungsbeispielen dargestellt sind, miteinander kombiniert und / oder ausgetauscht werden, ohne den Bereich der Erfindung zu verlassen.

## Patentansprüche

1. Verfahren zur Erzeugung von Biogas in einem Feststofffermenter (20, 22), in welchem stapelbare Biomasse von einem Perkolatstrom durchströmbar ist, um in einem Vergärprozess Biogas zu erzeugen,
**dadurch gekennzeichnet, dass**
der Vergärprozess anhand der Qualität des erzeugten Biogases geregelt wird,
wobei der Vergärprozess über die Regelung mindestens eines Betriebsparameters des Feststofffermenters (20, 22) geregelt wird,
wobei als Betriebsparameter die Temperatur in dem Feststofffermenter (20, 22) über die Perkolattemperatur, die Heiztemperatur des Perkolats, die Perkolationsdauer, die Perkolationsfrequenz, die Menge des aufgebrachten Perkolats und/oder der pH-Wert des Perkolats geregelt wird, und
wobei der Betriebsparameter als Stellgröße dient.

2. Verfahren gemäß Anspruch 1, **dadurch gekennzeichnet, dass** das Biogasvolumen, der Biogasvolumenstrom, der Methangasgehalt und/oder der Restgasgehalt des erzeugten Biogases bestimmt wird und anhand des jeweils bestimmten Parameters der Vergärprozess geregelt wird, und der Parameter bevorzugt als Regelgröße dient.

3. Verfahren gemäß einem der vorstehenden Ansprüche, **dadurch gekennzeichnet, dass** die Verweildauer der stapelbaren Biomasse in dem Feststofffermenter (20, 22) anhand der Qualität des erzeugten Biogases geregelt wird.

4. Verfahren gemäß einem der vorstehenden Ansprüche, **dadurch gekennzeichnet, dass** bei der Regelung des Vergärprozesses die Zusammensetzung, die Menge, die bisherige Verweildauer und/oder der Vergärzustand der in dem Feststofffermenter (20, 22) aufgenommenen Biomasse berücksichtigt wird, und bevorzugt eine Führungsgröße auf dieser Grundlage bestimmt wird.

5. Verfahren gemäß einem der vorstehenden Ansprüche, **dadurch gekennzeichnet, dass** für jeden Feststofffermenter (20, 22) in einer Biogasanlage (1) die Regelung des Vergärprozesses separat und unabhängig von den anderen Feststofffermentern (20, 22) durchgeführt wird.

6. Verfahren gemäß einem der vorstehenden Ansprüche, **dadurch gekennzeichnet, dass** mindestens zwei Feststofffermenter (20, 22) gleichzeitig aufgrund der Qualität des miteinander vermischten Gasstroms aller Biogasströme geregelt werden.

7. Verfahren zur Erzeugung von Biogas in einem Feststofffermenter (20, 22), in welchem stapelbare Biomasse mit Perkolat beaufschlagt wird, um in einem Vergärprozess Biogas zu erzeugen,
**dadurch gekennzeichnet, dass**
die Beaufschlagung der Biomasse mit Perkolat anhand der Qualität des erzeugten Biogases geregelt wird, wobei anhand der Qualität des erzeugten Biogases die Perkolationsdauer, die aufgebrachte Perkolatmenge und/oder die Perkolationsfrequenz geregelt werden.

8. Vorrichtung (1) zur Erzeugung von Biogas, umfassend mindestens einen Feststofffermenter (20, 22) zur Aufnahme stapelbarer Biomasse, wobei die stapelbare Biomasse von einem Perkolatstrom durchströmbar ist, um in einem Vergärprozess Biogas zu erzeugen,
**dadurch gekennzeichnet, dass**
eine Regelung (84) zur Regelung des Vergärprozesses anhand der Qualität des erzeugten Biogases vorgesehen ist, wobei die Regelung (84) auf die Temperatur in dem Feststofffermenter (20, 22) über die Perkolattemperatur, die Heiztemperatur des Perkolats, die Perkolationsdauer, die Perkolationsfrequenz, die Menge des aufgebrachten Perkolats und/oder den pH-Wert des Perkolats als Stellgröße wirkt.

9. Vorrichtung (1) gemäß Anspruch 8, **dadurch gekennzeichnet, dass** ein Sensor (80, 82) zur Bestimmung der Qualität des aus einem Feststofffermenter (20, 22) abgezogenen Biogases vorgesehen ist und die Regelung (84) die Perkolierung des Feststofffermenters (20, 22) regelt.

## Claims

1. A process for the production of biogas in a solid fermenter (20, 22) in which stackable biomass can be flowed through by a percolate stream to produce biogas in a fermentation process,
**characterized in that**
the fermentation process is controlled by the quality of the biogas produced, wherein the fermentation process is controlled by controlling at least one operating parameter of the solid fermenter (20, 22), wherein as operating parameter the temperature in the solid fermenter (20, 22) is regulated by means of the percolate temperature, the heating temperature of the percolate, the percolate period, the percolate frequency, the amount of applied percolate and / or the pH of the percolate, and wherein the operating parameter serves as a actuating variable.

2. The method according to claim 1, **characterized in that** the biogas volume, the biogas volume flow, the methane gas content and / or the residual gas content of the biogas produced is determined and is regulated by the particular parameter of the fermentation process, and the parameter is preferably used as a actuating variable.

3. The method according to any one of the preceding claims, **characterized in that** the residence time of the stackable biomass is controlled in a solid fermenter (20, 22) based on the quality of the biogas produced.

4. The method according to any one of the preceding claims, **characterized in that** in the regulation of the fermentation process, the composition, the amount, the previous residence time and / or the fermentation state of the biomass stored in the solid fermenter (20, 22) is taken into account, and preferably a reference variable is determined on this basis.

5. The method according to any one of the preceding claims, **characterized in that** for each solid fermenter (20, 22) in a biogas plant (1), the control of the fermentation process is carried out separately and independently of the other solid fermenter (20, 22).

6. The method according to any one of the preceding claims, **characterized in that** at least two solid fermenters (20, 22) are controlled simultaneously due to the quality of the mixed gas flow of all biogas streams together.

7. A method for producing biogas in a solid fermenter (20, 22), in which stackable biomass is applied with percolate to produce biogas in a fermentation process,
**characterized in that**
the application of biomass with percolate is determined based on the quality of the biogas produced, wherein based on the quality of the biogas produced, the percolation period, the amount of applied percolate and / or the percolation frequency is regulated.

8. Device (1) for the production of biogas, comprising at least one solid fermenter (20, 22) for receiving stackable biomass, wherein the stackable biomass can be flowed through by a percolate stream to produce biogas in a fermentation process,
**characterized in that**
a control unit (84) is provided for controlling the fermentation process based on the quality of the biogas produced, wherein the control unit (84) acts on the temperature in the fermenter on the basis of the actuation variable in form of the percolate temperature (20, 22), the heating temperature of the percolate, the percolation time, the percolation frequency, the amount of applied percolates and / or the pH of the percolate.

9. Device (1) according to claim 8, **characterized in that** a sensor (80, 82) for determining the quality of biogas removed from a solid fermenter (20, 22) is provided and the control unit (84) controls the percolation of the solid fermenter (20, 22).

## Revendications

1. Procédé de production de biogaz dans un fermentateur de solides (20, 22), dans lequel une biomasse empilable peut être traversée par un courant de percolat pour produire du biogaz dans un processus de fermentation,
**caractérisé en ce que**
le processus de fermentation est réglé sur la base de la qualité du biogaz produit,
dans lequel le processus de fermentation est réglé par la régulation d'au moins un paramètre opérationnel du fermentateur de solides (20, 22),
dans lequel, en tant que paramètre opérationnel, la température dans le fermentateur de solides (20, 22) est réglée par la température du percolat, la température de chauffage du percolat, la durée de percolation, la fréquence de percolation, la quantité de percolat collectée et/ou le pH du percolat, et
dans lequel le paramètre opérationnel sert de grandeur de réglage.

2. Procédé selon la revendication 1, **caractérisé en ce que** le volume de biogaz, le courant volumique de biogaz, la teneur en gaz méthane et/ou la teneur en gaz résiduel du biogaz produit est déterminé(e) et sur la base du paramètre déterminé respectif, le processus de fermentation est réglé, et le paramètre sert de préférence de grandeur de réglage.

3. Procédé selon l'une des revendications précédentes, **caractérisé en ce que** la durée de séjour de la biomasse empilable dans le fermentateur de solides (20, 22) est réglée sur la base de la qualité du biogaz produit.

4. Procédé selon l'une des revendications précédentes, **caractérisé en ce que,** lors du réglage du processus de fermentation, la composition, la quantité, la durée de séjour jusque-là et/ou l'état de fermentation de la biomasse reçue dans le fermentateur de solides (20, 22) est pris(e) en compte, et de préférence une grandeur de guidage est déterminée sur cette base.

5. Procédé selon l'une des revendications précédentes, **caractérisé en ce que** pour chaque fermentateur de solides (20, 22) dans une installation de biogaz (1), le réglage du processus de fermentation est réalisé séparément et indépendamment des autres fermentateurs (20, 22).

6. Procédé selon l'une des revendications précédentes, **caractérisé en ce qu'**au moins deux fermentateurs de solides (20, 22) sont réglés en même temps sur la base de la qualité du courant de gaz mélangé conjointement de tous les courants de biogaz.

7. Procédé de production de biogaz dans un fermentateur de solides (20, 22), dans lequel une biomasse empilable est alimentée avec un percolat, pour produire du biogaz dans un processus de fermentation,
**caractérisé en ce que**
l'alimentation de la biomasse avec le percolat est réglée sur la base de la qualité du biogaz produit, dans lequel sur la base de la qualité du biogaz produit, la durée de percolation, la quantité de percolat collectée et/ou la fréquence de percolation sont réglées.

8. Dispositif (1) pour la production de biogaz, comprenant au moins un fermentateur de solides (20, 22) pour la réception de biomasse empilable, dans lequel la biomasse empilable est traversée par un courant de percolat, pour produire du biogaz dans un processus de fermentation,
**caractérisé en ce que**
un réglage (84) pour le réglage du processus de fermentation est prévu sur la base de la qualité du biogaz produit, dans lequel le réglage (84) agit sur la température dans le fermentateur de solides (20, 22) par le biais de la température de percolat, la température de chauffage du percolat, la durée de percolation, la fréquence de percolation, la quantité du percolat collecté et/ou le pH du percolat en tant que grandeur de réglage.

9. Dispositif (1) selon la revendication 8, **caractérisé en ce qu'**un capteur (80, 82) est prévu pour la détermination de la qualité du biogaz retiré d'un fermentateur de solides (20, 22) et le réglage (84) règle la percolation du fermentateur de solides (20, 22).
